# EUROPEAN PATENT SPECIFICATION

(11) **EP 2 851 820 B1**
(45) Date of publication and mention of the grant of the patent: **02.09.2020**
(21) Application number: 13185298.0
(22) Date of filing: 20.09.2013
(51) Int. Cl.: G16H 40/60

(54) **Measurement data processing method and apparatus**
Verfahren zur Messdatenverarbeitung und Vorrichtung
Procédé de traitement de données de mesure et appareil

(43) Date of publication of application: 25.03.2015
(73) Proprietor: FUJITSU LIMITED, Kawasaki-shi, Kanagawa 211-8588 (JP)
(72) Inventor: Li, Zhaojun, Guildford, Surrey GU3 2DJ (GB); Hisatomi, Makiko, Amersham, Buckinghamshire HP6 6FH (GB)
(74) Representative: Haseltine Lake Kempner LLP

(56) References cited:
- WO-A2-2005/114524
- US-A1- 2004 030 581
- US-A1- 2004 117 204
- US-A1- 2011 172 550
- US-B1- 7 292 956

## Description

This invention relates to a method and apparatus for processing measurement data received from a remote device monitoring a subject, particularly in the field of healthcare.

Healthcare in the community (including home care/out-patient care), instead of in-patient and intensive care, is becoming a global trend in order to reduce healthcare expenditure and to improve patient experience. Continuous or frequent measurements may be needed to be taken in patients' normal everyday life in addition to one-stop measurement at clinics or hospitals, especially for chronic disease management. Such one-stop measurements are usually carried out by experienced healthcare professionals. If condition changes are notified to the clinical organisations, early detection can potentially prevent degradation of patients' conditions and save cost associated with further treatment. Especially for people living alone, detection of condition change is critical in emergency cases (e.g. falling down, stroke, heart attack).

One of the main challenges associated with healthcare in the community is the increased responsibility for the patients. The patients will play an increasing role in managing their health, compared to current healthcare delivery model in which the patients rely on healthcare professionals such as doctors and nurses for their health management. For example, instead of staying in a hospital where nurses come around to take blood pressure and temperature measurements, patients may stay at home and they will be responsible for collecting the measurements remotely and sending them to appropriate healthcare providers. However, the patients may not necessarily understand the implication of the health data. Therefore, if they are not collecting the data appropriately (e.g. a device was not attached correctly), they might not be able to notice the difference. If the data are incorrectly collected, the patients have to collect the data again, which would potentially waste both the patients' and doctors' time. Therefore, it would be very useful to have a mechanism to detect errors and abnormalities in measurement data collection and preferably to notify the errors to the patients and the doctors.

US 7 292 956 B1 discloses a method and system for management of medical data from a network of devices including federated sensors that collect and forward medical data pertaining to an individual or biological specimen. The federated sensors or a central or remote device further process the medical data and assign a priority value to the medical data and processing can also include the analysis of the data for sensor error, local fusion of multiple sensors into higher-level interpretations, and the summarization or abstraction of the data into information that people are more comfortable with sharing than they might be with transmittal of the base data.

With the foregoing in mind, it is desirable to help the healthcare professionals accept remote data.

According to an embodiment of a first aspect of the invention, there is provided a computer-implemented method of processing measurement data received from a remote device monitoring a subject, the method comprising: comparing the measurement data against an expected data profile (EDP) for the subject, the EDP being derived from historical measurement data and comprising configuration information as to the configuration of the measurement data and expected value information as to the expected values of the measurement data; inputting measurement data relating to different health readings and adding the input measurement data to the EDP to form an overall EDP which takes into consideration the different health readings and any data correlation between the health readings; marking the measurement data with an error tag if it does not match the data correlation maintained in the EDP and included in the configuration information; marking the measurement data with an abnormal tag if it does not match the expected value information; and subsequently assessing any measurement data not marked with an error tag or abnormal tag, by excluding the measurement data marked with an error tag or abnormal tag from the assessment, to check whether it indicates a health condition change of the subject, wherein the configuration information indicates the device identity and/or frequency of receipt and/or amount and/or type of the management data and/or data size per transmission.

Error detection in health measurement is difficult, as a change in the collected measurement data might not be an error, but rather a reading related to a health condition change. In order to detect errors due to faulty devices, currently readings can be manually carried out by professionals (i.e. doctors, trained nurses or other healthcare professionals) to allow device (including sensor) calibration. However, currently the changes in the monitored data at home may not be notified in real-time to the doctors, with any indication as to whether a change is (1) an error or (2) a change in health condition. This leads to the problem that the doctors do not know whether the remote data is correctly collected until the patient's (subject's) next visit to the doctor. This further leads to the more serious problem that the change in health condition may not be identified by the doctors until much later, and the conditions can get worse during that time. Therefore, these patients tend to stay in the hospitals longer to ensure accurate and timely health condition change alert/identification, as there are no appropriate mechanisms to support this condition change alert remotely.

If a condition change is notified to doctors/nurses/IT support etc. earlier, this can provide intervention points for them for both cases. For the case (1) with wrong implementation, the patients may be told that they are not using the equipment properly. For the case (2), the doctors/nurses can take a closer look at the reading (or increase the measurement frequency), as a condition change has been identified.

As patients are expecting much better patient experience in the future, it is important not to waste their time and effort. By ensuring that their effort will be contributing to combating the health problem together with the doctors, wasted time and effort can be reduced. Further, the method described above can involve automatic assessment of measurement data such that potential health condition changes are identified promptly.

On the doctor side, a cultural change may be required to accept data collected by patients. Currently doctors do not necessarily trust the accuracy/quality of the data collected by a patient. With increasing healthcare demands, it is helpful for the doctors to be able to focus on more complicated and pressing issues, rather than worrying about the quality of collected data.

The inventors have come to the conclusion that what may help the doctors to trust the collected data, and use them as part of their patient consultation, is if they can be assured of the quality of data. If the patient-collected data is of a similar standard to their own measurements this may help doctors to trust the data. One way to achieve this is by calibrating measurement data against a reference. Data may for example be the combination of several health readings, e.g. body temperature and blood pressure and the checking of collected data may involve comparing collected data against pre-defined criteria, which criteria are set by a healthcare professional for each individual patient.

Therefore an important issue to be addressed is how to perform remote calibration of measurement data collected from sensors/devices, so that changes (either errors or changes in health condition, for example) can be further identified in the collected measurement data and the appropriate people notified. This may include categorisation of the changes, in order that healthcare professionals may readily identify what types of changes have occurred. Alternatively, the healthcare professionals may themselves categorise a specific type or similar group of changes.

The present invention uses comparison with an EDP as a calibration.

The EDP includes configuration information and expected value information. Configuration information as used herein may be taken to include information relating to the current configuration of one or more devices for a given patient (e.g. frequency of measurement reporting per day). Configuration information may also include the characteristics of the device which can be found from manufacturer's specification. Device characteristics may thus indicate characteristics of the device measurement data, and preferably include for example data size per transmission, device sampling rates, type and level of detail of data etc. One example of a scenario when measurement data is not considered to match the configuration information is missing data, which occurs for example when no data is received at an expected time. Data received at the wrong time would also indicate that the configuration information and the measurement data do not match. If data is received in the wrong format, for example if the wrong model of device is used, or simply incorrectly programmed, this could also result in a non-match. Further examples include when data packets received are too big, or too small. This may indicate that a remote device is not configured appropriately.

One example EDP for a patient using such a remote device could include the following: healthcare sensor/device information (e.g. configuration parameters), the patient's measurement profile (e.g. report configuration, measurement frequency, etc.) and expected measurement data (e.g. in the form of a range, or threshold) for specific categories of data, such as blood pressure, temperature, ECG (electrocardiogram), etc. In this example the first two parts of the EDP relate to the condition in which the data is expected to be received and the last part of the EDP relates more to the main content of the profile. Different healthcare sensors/devices may be used for different patients. The performance/quality of such devices varies, which may affect the collected health readings. Therefore healthcare sensors/devices information can be an important component of the EDP for calibration purposes. Similarly, a patient's measurement profile may also vary depending on the health condition. Such profile may include measurement frequency, report configuration, which may help with building up the EDP as well as detecting abnormal data readings.

Expected value information as used herein may be taken to include information indicating an expected value of the measurement data for the patient and/or a representative patient or a group of patients. This information may be based on past measurements for which no significant condition change is expected.

An example of data which is considered as erroneous could occur when the data received does not meet the current configuration, e.g. the amount of data received is less than what was expected based on current measurement and report configuration. If the received data does not meet the data correlation maintained in the EDP, it may be considered as "abnormal". For example, the temperature range of a certain patient may normally be correlated to a range of blood pressure readings. So if a set of measurement data is received where the temperature is within a specified temperature range but the pressure is outside of a specified pressure range, then this reading may be considered abnormal.

Alternatively, if a change is expected it may be based on the expected amount or path of the change, and a deviation therefrom. Expected value information may also include information relating to a category of data in which the data is expected to remain constant, i.e. with no significant change, in combination with another category of data expected to indicate a change. For example, if a patient undergoes a procedure which is only known to affect certain measurement data, a 'stable' value/category could be used to indicate an abnormal reading, rather than a changed value in the data which is expected to be affected.

An expected data profile (EDP) may provide a history of a subject, such as a patient of a healthcare service, being monitored and may contain data relating to many different categories. For example, in this scheme, for each patient/user of the healthcare services an EDP is maintained. This EDP may for example be maintained in the Cloud (computing capacity provided in a communication network, probably using an application executing on a server). Based on the history of the patient's data, this EDP can be calculated from the healthcare device's information (e.g. configuration parameters) and patient's measurement profile (e.g. measurement frequency, report configuration, etc.).

In order to build up the EDP for remote calibration, it is desirable to have reference readings of that particular patient which give an understanding of possible differences in measurement. This can be achieved for example by having at least two sets of measurement data to compare, one of which has been measured by a doctor with his/her usual trusted equipments (preferably with a higher level of accuracy), and the other which has been measured by the patients with the provided remote monitoring devices. This difference may decide the range of tolerance of the device measurement that should to be taken into account when building up the EDP. These reference readings can be collected regularly at the time of face-to-face meetings between the patient and the doctors/trained nurses, for example at the time when patients and devices are registered or updated, as well as each time the doctors and/or trained nurses perform "on-the-spot" calibration of the devices. "On-the-spot" calibration of the sensors/devices may refer to a case where a doctor and/or trained nurse calibrate the sensors/devices by comparing the measurement data collected by a doctor or a trained nurse with his/her usual trusted equipment (i.e. which would likely have a higher level of accuracy), with the data measured by the patients with the provided remote monitoring devices.

Another part of building up an EDP for remote calibration and data tagging is that the health readings (e.g. blood pressure, temperature, ECG, etc.) can only be judged to a certain level of accuracy without understanding particular patient's condition. Therefore it is not adequate to just treat one parameter separately to identify the problem. The EDP can be built upon a history of the patient's data as well as the correlation of several health measurements (e.g. the temperature and the associated blood pressure for a particular patient in a particular health condition). This allows for comparison of the relative parameter changes relating to the patient rather than the absolute value of the parameters. This more-complete picture of a patient's condition may further serve to increase doctor confidence in the devices. For example, if one parameter relating to a patient changes, this could have the implication of a particular type of condition change of that patient. If the same change occurs, but is taken in combination with a change of another (category of) parameter, then a more accurate diagnosis may be reached. This can lead to a different type of condition change being identified, than with one parameter only.

Although the example given here is related to healthcare implementation, the remote measurement calibration and tagging mechanism of invention embodiments can be implemented in other scenarios for calibrating measurement data and providing additional information about the data. For example, it can be used for smart metering application at home. Smart metering may involve the use of remote devices located in or around household appliances.

Taking a household boiler as an alternative example, remote devices may be attached inside the boiler to monitor average temperature, rusting, water purity, etc. Further devices may be located elsewhere, such as at the in- or outlet of the boiler in order to monitor average usage. The "expert" in this case would likely be a plumber or technician. Thus the remote devices may produce measurement data which can be monitored remotely. The health condition of a boiler in the context of this invention may be considered to include the age, efficiency and general maintenance of the boiler. A condition change could for example occur due to scaling build up inside the boiler, etc. An EDP in this case may include historical measurement data taken from each remote device, a service history of the boiler and manufacturer specifications. In this case, data recorded for other, similar boilers (similar models for example) could also be used to build up a still more detailed EDP.

In healthcare and other scenarios, when a set of measurement data arrives (is input for processing from the remote device location), for example in the Cloud, the received data is checked against the EDP and any appropriate tag put on the received data, for example in order to provide additional information for healthcare professionals to make decisions based on the data.

Preferably, the method of processing management data further comprises accepting expert input when creating the EDP. Additionally or alternatively expert input may be used when assessing whether the measurement data indicates a health condition change of the subject.

An expert may be any person with sufficient knowledge to interpret the collected management data. For example, an expert may be a doctor, nurse or other healthcare professional. An expert may also be an IT (information technology) specialist or computer programmer. In the alternative example given above, the expert may be a plumber or technician. The expert preferably has the ability to set up and modify the EDP (for example to register the user and the user's device(s) and/or to set medical thresholds and/or to input an initial data set), and to override measurement data and/or the EDP by manual input/correction, which may be useful in a situation where the doctor wishes to adjust or label the measurement data to highlight a particular aspect of the measurement data and/or an aspect of the patient's activities in order to isolate an particular condition. The expert may be involved in each determination of whether the measurement data includes a health condition change or involved only if the measurement data satisfies one or more criteria (such as thresholds).

Advantageously, the method of processing management data further comprises marking the measurement data with a health condition change tag if the measurement data indicates a health condition change. The health condition change tag may indicate a type of the health condition change. In addition, certain medical thresholds may be defined by health professionals for individual patients, which can be used to decide if a measurement reading indicates a possible health condition change. This information is also a useful component of the EDP. Types of health condition change can range from very broad definitions to very specific, depending on the change. Doctors or other experts may wish to categorise the various changes based on the type of change, by making custom names for the changes and/or by selecting a number of changes to be designated as one 'type'. This may further assist the doctor when reviewing a patient's condition, as they will be familiar with the historical data.

If the measurement data indicates a health condition change the method may further comprise generating an alert indicating the health condition change of the subject. Further, the method may also include transmitting the alert, wherein the transmission destination is selected in dependence upon the nature of the health condition change and/or a user setting. Alerts may be sent to patients/clinical staff/IT support/family members, depending on the nature of the alerts and particular user settings. One example could be based on the agreement, an alert could be sent to the mobile phone of a family member of the elderly patient. Depending on the urgency of the alert, for example in case of emergency, the alert may be sent to healthcare/medical personnel immediately. The generation of an alert may serve to indicate urgency related to the health condition change, such as for example a trip or fall of a patient or an irregular heart beat. An alert may be in the form of a message or indicator capable of being generated quickly and transmitted to an appropriate healthcare professional, such as a patient's general practitioner or a first response (first aid) officer. The alert may contain additional information relevant to the nature of the health condition change, such as geographic location so that the patient may be located in the event of a serious condition. Additionally the alert may cause the device to increase the frequency of data transmission or even continuously transmit, for example in the event of a patient suffering a heart attack, so that paramedics or similar may have advanced knowledge of the patient's condition before arriving at the patient's location.

User settings may be set to ensure the alert is sent (additionally or alternatively) to a designated location or person regardless of the condition or dependant on the condition. An example of this would be if a patient wishes a close friend or family member to be contacted, perhaps because they live in the proximity of the patient and may be able to respond to the alert quickly in the event the patent undergoes a serious condition change. A patient may also simply wish to keep a selected person apprised of their condition.

Preferably, the EDP includes configuration information and/or expected value information in the form of a range; and the measurement data must fall within the range to match the configuration information or the expected value information.

The method of processing measurement data may include setting boundary conditions or values (a maximum and minimum, for example) for the measurement data in order to define a range such that the measurement data must fall within the range in order to match the configuration information or the expected value information. This method may be used for data types which vary across a range, such as blood pressure or heart rate, etc. It may be beneficial if certain medical thresholds are defined by doctor(s) for individual patients, which can be used to decide if the measurement reading indicates the possible health condition change.

The method of processing measurement data may further comprise recalibrating the configuration information and/or the expected value information based on the measurement data. As the EDP is built up over time, the boundary conditions and/or data making up the EDP may be changed (adapted) to a patient's individual needs. For example, if a patient's condition changes significantly and the boundary conditions and/or data are no longer appropriate or (diagnostically) relevant, they may be changed (either automatically, or manually by the expert) to give more meaningful readings.

The method of processing measurement data may further comprise recalibrating the configuration information and/or the expected value information, wherein the recalibrating involves updating the range by taking the measurement data into account. By recalibrating the configuration information and/or the expected value information more useful readings can be obtained and implications of those readings and/or changes thereto can be more readily identified. It may also be appropriate to cancel ranges relating to a particular type of data. For example, if data of a certain type is only significant to the patient's condition when it exceeds an upper or a lower limit, a range may be replaced with a simple threshold for the condition where appropriate.

The method of processing measurement data may further comprise adding the measurement data to the historical data from which the expected data profile is derived.

In order that a more complete picture of a patient's condition may be obtained, historical data may be continuously grown to include each set of measurement data received. Thus the EDP for each patient may become more accurate and detailed over time.

The configuration information is not necessarily limited to any particular category of information. Configuration information may include information advised, by the manufacturer of a remote device, as being relevant and/or may include information which a healthcare professional considers relevant. For example, a manufacturer may be aware of certain limitations associated with a particular remote device and may consider this noteworthy for a healthcare professional when interpreting measurement data. Conversely, a healthcare professional may have experience using a particular remote device and may for example consider that information relevant.

It may be useful to combine the input measurement data, which may be obtained from multiple remote devices. By combining this data, a more complete picture of a patient's condition can be obtained. For example, a patient may use a pedometer as a remote device to monitor activity. In addition to this, they may consider wearing a portable heart rate monitor in order to obtain a more complete set of data relating to the fitness of the patient, and a correlation between movement and heart rate. In this way the EDP may be built up not only on a history of measurement data but also on the correlation between several health measurements.

The method of processing measurement data may further comprise inputting measurement data from an expert and adding the input measurement data to the EDP.

It may be advantageous to allow healthcare professionals the ability to add data, which may be generated using far more accurate equipment and/or taken under specific circumstances, in order to provide a more complete picture. This may assist the doctor when diagnosing a patient, because the higher accuracy and/or controlled environment may allow the doctor to eliminate environmental and other factors.

The method of processing measurement data may further comprise performing diagnostics on measurement data marked with an abnormal tag to determine a type of abnormality. This may further help to analyse and diagnose a type of abnormality so that similar abnormalities can be more readily recognised in future data received. For example a particular condition may be regarded as being abnormal in general until it is analysed and categorised. If subsequent similar abnormalities of the same category are then detected, this can be useful in highlighting trends and/or inconsistencies in the diagnosed condition relating to the abnormality.

According to an embodiment of a second aspect, the invention provides an apparatus for processing measurement data received from a remote device monitoring a subject, the apparatus comprising: a memory storing an expected data profile (EDP) for the subject, the EDP being derived from historical measurement data; an expected result manager configured to compare the measurement data against the EDP, the EDP comprising configuration information as to the configuration of the measurement data and expected value information as to the expected values of the measurement data for that subject, and to input measurement data relating to different health readings and add the input measurement data to the EDP to form an overall EDP which takes into consideration the different health readings and any data correlation between the health readings; a tag generator configured to mark the measurement data with an error tag if it does not match the configuration information, and mark the measurement data with an abnormal tag if it does not match the data correlation maintained in the EDP and included in the expected value information; and a data analyser configured to assess any measurement data not marked with an error tag or abnormal tag, by excluding the measurement data marked with an error tag or abnormal tag from the assessment, to check whether it indicates a health condition change of the subject, wherein the configuration information indicates the device identity and/or frequency of receipt and/or amount and/or type of the management data and/or data size per transmission.

The apparatus may also comprise input means for the measurement data from one or more remote device and output means for processed data and/or alerts. The processed data may be sent to a hospital or other care institute. The apparatus may be linked to or comprise a user interface for expert input.

Any and all features of the different aspects can be combined since they relate to the same invention. Features and sub-features of one aspect may be applied to any of the other aspects.

In any of the above aspects, the various features may be implemented in computer hardware, firmware, software, cloud-based platforms or in combinations of them. The invention can be implemented as a computer program or computer program product, i.e., a computer program tangibly embodied in an information carrier, e.g., in a machine-readable storage device or in a propagated signal, for execution by, or to control the operation of, one or more hardware modules.

A computer program can be in the form of a stand-alone program, a computer program portion or more than one computer program and can be written in any form of programming language, including compiled or interpreted languages, and it can be deployed in any form, including as a stand-alone program or as a module, component, subroutine, or other unit suitable for use in a data processing environment. A computer program can be deployed to be executed on one module or on multiple modules at one site or distributed across multiple sites and interconnected by a communication network.

Method steps of the invention can be performed by one or more programmable processors executing a computer program to perform functions of the invention.

Processors suitable for the execution of a computer program include, by way of example, both general and special purpose microprocessors, and any one or more processors of any kind of digital computer. Generally, a processor will receive instructions and data from a read-only memory or a random access memory or both.

The invention is described in terms of particular embodiments. Other embodiments are within the scope of the following claims. For example, the steps of the invention can be performed in a different order and still achieve desirable results. Thus the error tagging may take place after the abnormal reading tagging in one embodiment. Performing error tagging before abnormal reading tagging has the benefit of removing false or erroneous measurement data before the data is analysed to determine if the reading is abnormal or indicates a health condition change. Performing the abnormal tagging first is less usual but may provide the benefit that the abnormal tagging may be performed on the entire set of, thus far unprocessed, collected data.

The skilled person will appreciate that elements of the invention may be spatially separate but can combine to serve the functions defined. Equally, the same physical parts of the invention may provide two or more of the functions defined.

A method according to preferred embodiments of the present invention can comprise any combination of the previous apparatus aspects.

Preferred embodiments of the invention will now be described, by way of example only, with reference to the accompanying drawings in which:
Figure 1 is a flowchart of measurement data processing according to an embodiment;
Figure 2 is a block diagram showing an apparatus for processing measurement data according to an embodiment;
Figure 3 is an example of an end-to-end system in which the present invention may be implemented;
Figure 4 is a flowchart of measurement data processing according to another embodiment; and
Figure 5 is a block diagram showing an apparatus for processing measurement data according to another embodiment.

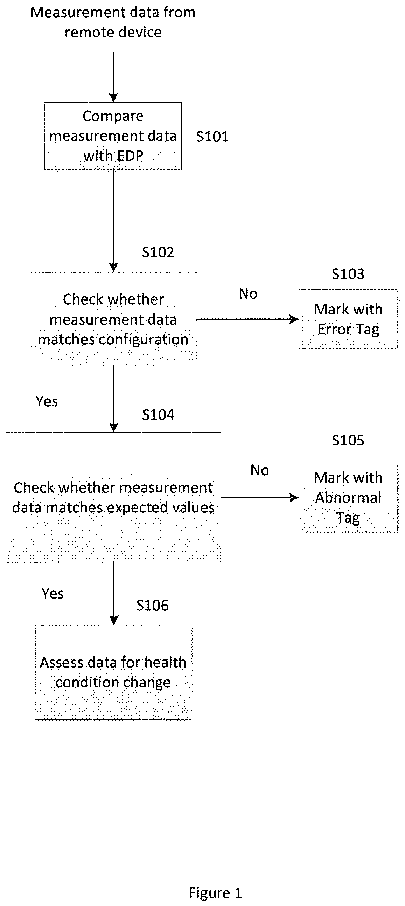

Figure 1 is a simplified flowchart of a method of measurement data processing according to a general invention embodiment. Measurement data is received from a remote device. The measurement data relates to measurements taken by the remote device and may be communicated over a wireless/wired or other network for processing. The remote device may be any device used by a patient or user, which serves to monitor the patient or user in some way, particularly in the field of healthcare. For example, the remote device may be a heart monitor or activity monitor, which is able to take periodic measurements relating to the patient's condition. The measurement data is then compared with an Expected Data Profile (EDP). The EDP contains data which may be based primarily on a patient's own historical data possibly expanded with the statistics of the measurement data and data correlation, for example between measurement data for different healthcare readings. The statistics of the measurement data include, for example, the readings of a patient's temperature, blood pressure, etc., which could be based on the patient's medical record. The correlation of the measurements is derived from these readings; for example, for a patient with certain condition a certain range of body temperatures generally correspond to a certain range of the readings of the blood pressure of this patient. If the received data does not meet the data correlation maintained in the EDP, it may be considered as "abnormal". For example, if the temperature range (T1, T2) of a certain patient is normally correlated to the range of blood pressure (P1, P2) reading and the temperature reading T falls within a designated "normal" range (T1, T2), but the blood pressure reading P falls outside the "normal" range (P1, P2), then this reading may be considered abnormal. The EDP may be influenced by many factors, such as expert input from a trained healthcare professional (expert), or other specialist. Such other specialists could for example include an electronics specialist who has experience with remote devices. By comparing measurement data with the EDP, or analysing the measurement data based on the EDP, it may be possible to identify incorrect data readings in the collected measurements. The results of the comparison may allow the measurement data to be marked with an appropriate tag(s). This has the benefit of providing more information for healthcare professionals (doctors/trained nurses) to make professional decisions.

An example of an abnormal reading may be a body temperature of 10 degrees centigrade (°C); while a reading of 40 degrees centigrade (°C) may merely indicate a condition change. The settings of the thresholds are therefore very useful for an EDP. Thus, depending on the setting of the thresholds, some "abnormal readings", or readings which appear initially to be abnormal, may also indicate some sort of health condition change.

As shown in figure 1, in one embodiment the received measurement data is compared with configuration information (S101). A check is performed to see whether the measurement data is deemed to "match" the configuration information (S102), for example in terms of data size per transmission. If the measurement data is considered to match the configuration information, then the measurement data is not tagged (S102 - "Yes"). However, if the measurement data is not considered to match the configuration information (S102 - "No"), then the measurement data may be marked with a tag (S103), for example with an error tag indicating that an error exists.

Measurement data is not necessarily required to be identical to the configuration information. In a practical sense, the two data/information sets may be taken or set under different circumstances and so a level of variation between the two sets is to be expected. For example, configuration information, when set, might not take into account a remote device user's individual circumstances, which may lead to variation between the configuration information and the measurement data. Therefore an amount of variation may be set before the measurement data is deemed not to match the configuration information. This may be tailored to each user's needs.

In the embodiment shown in figure 1, when measurement data is determined not to match the configuration information, the measurement data is tagged. This tag may be any form of indicator to make a healthcare professional, or others, aware that the tagged data may be "erroneous" in some way. This would allow a healthcare professional, or technician, to easily find erroneous data amongst the measurement data, so that the data may be checked to determine the cause and/or type of the error. Tagging may also be used to exclude the tagged data from later analysis. Preferably the tagged data could thereafter either be re-introduced to the analysed measurement data, if the cause of the error can be determined and/or compensated for, or the tagged data may simply be excluded completely from any further analysis.

As shown in figure 1, in one embodiment the received measurement data is then compared with expected value information to determine whether they match (S104). Expected value information relates to the actual measurement data received. For example, the expected value information may match if the current value is within an acceptable margin from the historical value range of values. Put simply, expected value information gives an indication of what the received data should be, whereas configuration information gives an indication of how the data should be received, e.g. in what format and when. Therefore, if the measurement data is deemed not to match the expected value information, this may indicate abnormal measurement data. In the event the measurement data is deemed not to match the expected value information (S104 - "No"), the measurement data is marked with an abnormal tag (S105), indicating an abnormality in the data. There are many reasons why measurement data may be regarded as abnormal. The measurement data could be "abnormal", because of incorrect readings due to e.g. faulty devices/sensors, or it could be the indication of healthcare condition change (or non-change if a condition change was expected). Either case requires further investigation/diagnostic from professionals (doctors/nurses/IT support). Conversely, if the measurement data is deemed to match the expected value information (S104 - "Yes"), then no tag indicating an abnormality in the measurement data, is set.

In the embodiment shown in figure 1, once the measurement data has been processed, such that "erroneous" or "abnormal" data have been marked with appropriate tags, the measurement data (which may include the deemed erroneous and/or abnormal data, possibly depending on the result of the further diagnostics carried out thereon) may be assessed in order to determine whether the user has undergone a condition change (S106). In healthcare, this may indicate for example an improvement of a patient's condition or a decline thereof.

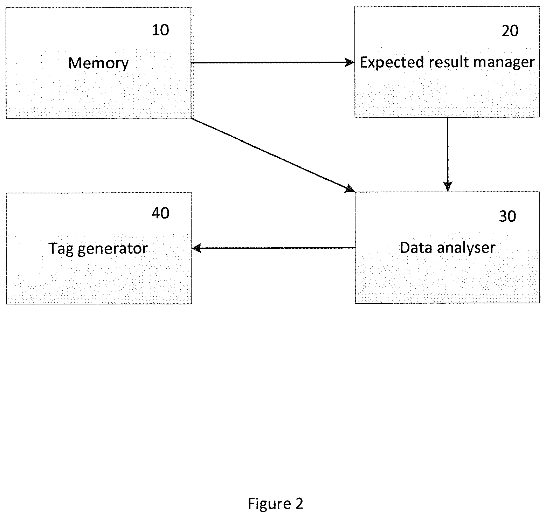

Figure 2 shows a simplified block diagram representing an apparatus for processing measurement data according to invention embodiments. In an embodiment of the invention, the apparatus includes a memory (data store) 10, an expected result manager 20, a tag generator 40 and a data analyser 30. In practice, these latter three parts may all be provided by the same appropriately programmed processor.

Data, preferably including received measurement data and a user's EDP, is stored in the memory 10. The expected result manager 20 creates and updates the EDP. The parameters in the EDP (such as frequency of data measured, expected volume of data per data transmission, timing of data receiving etc., for example) may be calculated by using device data, user measurement information and thresholds available in the memory 10. By comparing the data received and the parameters in an EDP (stored in expected result manager), the data analyser 30 is able to make decisions as to which tag should be used to mark received data. The method shown in figure 1 may be performed by the data analyzer 30. Based on the decisions made by the data analyzer 30, the tag generator 40 is arranged to generate tags.

The path followed by the arrows in figure 2 represents only one possible communication path. Alternative paths are envisaged, for example if data marked with an error tag are reviewed by a healthcare professional or similar and the data determined not to be erroneous. The data may be stored in the memory 10. The data or a derivative thereof is then subsequently re-transmitted to the data analyser as part of the EDP for use when making decisions on the tagging of data. The data, whether tagged or not, is preferably always stored in the memory 10, following each comparison and assessment cycle. Following each cycle, the untagged data is added to the appropriate EDP, stored in the memory 10.

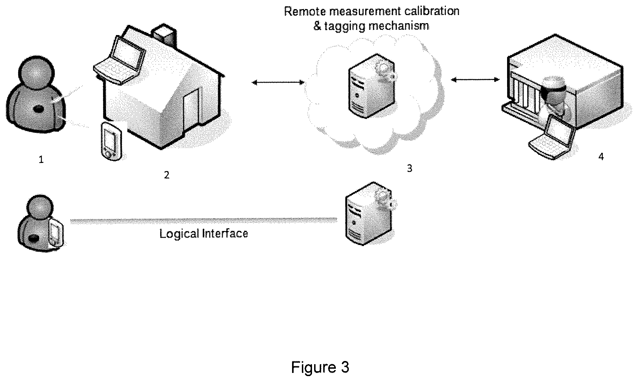

Figure 3 shows one possible system in which embodiments of the invention may be employed. In the system shown in figure 3, the collected (received) data (from healthcare sensors/devices) 1 are transferred wirelessly to a gateway 2 (such as a wireless router or home eNodeB) and then to the Cloud 3; and shared with doctors/nurses/IT support 4. In this way, healthcare professionals are able to access the data remotely. This remote calibration and tagging mechanism, in the Cloud, may be used to calibrate the data from healthcare measurements of a patient and generate alerts/tags for patients, doctors and others if necessary.

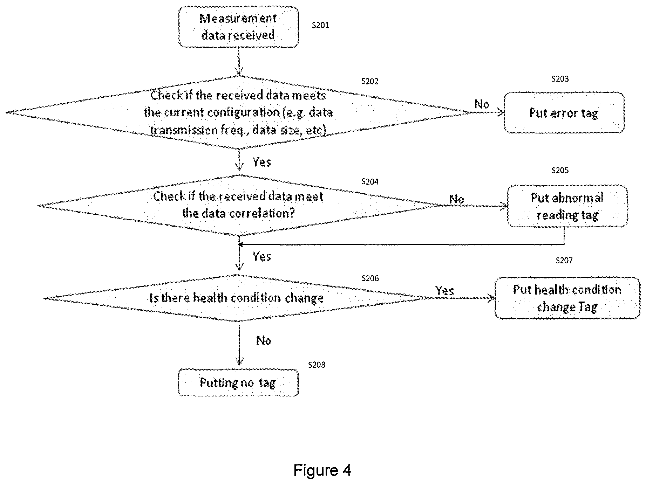

Figure 4 shows a flowchart of a method of measurement data processing according to another embodiment of the invention. The steps shown in figure 4 relate closely to those shown in figure 1, with an additional tagging step.

The steps shown in figure 4 include: receiving measurement data from a remote device (S201) and performing a check to see if the received data meets the current configuration (S202). The check may be, for example, whether the frequency and amount of the received data are in line with settings maintained in the EDP. If not (S202 - "No"), an "error" tag associated with the data may be set (S203), to indicate that the tagged data are erroneous. A reason for the erroneous measurements may be faulty devices/sensors, or incorrect attachment of the device/sensor has not been attached correctly, or packet loss caused by a poor transport network condition, for example. If the received data are in line with the settings, (S202 - "Yes") there is a check to see if the received data meet the data correlation maintained in the EDP (S204). If not (S204 - "No"), an "abnormal reading" tag associated with the data may be set (S205), which indicates that the reading is abnormal based on the data correlation and this patient's historical data. The "abnormal readings" could indicate incorrect readings due to, e.g. faulty devices/sensors, or could be the indication of healthcare condition change. Either case may require further investigation/diagnostic from the professionals (doctors/nurses/IT support) and thus the process shown in this example returns to a check for a health condition change (S206). In this case the check may be manual. If the received data meets the data correlation maintained in the EDP (S204 - "Yes"), there is also a check (which is more likely to be automated and based on the EDP) if the received data indicates a health condition change (S206). If so (S206 - "Yes"), a "health condition change" tag associated with the data may be set (S207), which shows that the reading may indicate a possible health condition change. If the data does not indicate a health condition change (S206 - "No"), no tag is set (S208).

The embodiment shown in figure 4 may be more beneficial when a substantial amount of measurement data has been collected such that the EDP is considered to reflect a patient's health condition to a relatively high level of accuracy. In these circumstances, measurement data containing a health condition change tag may be very useful to a doctor when diagnosing a patient. A doctor may be directed towards the data tagged with a health condition change tag, which may help the doctor to identify the change much sooner.

The health condition change tag may also indicate the type of health condition change, based on the historical data contained in the EDP. Data collected from other patients with similar conditions may also be used in order to identify trends and common symptoms amongst patients and/or differences therebetween. Therefore, the patient may effectively already have a preliminary diagnosis in relation to the change, which may be subsequently checked by the doctor.

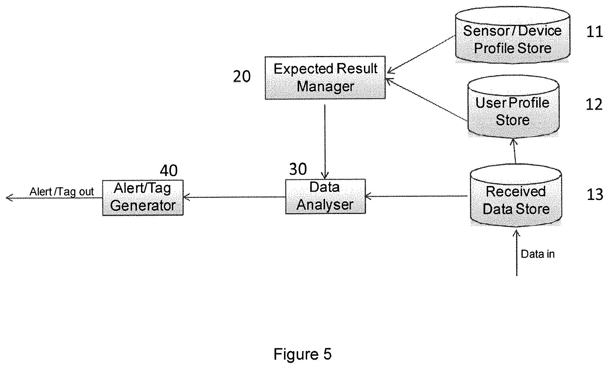

Figure 5 shows another exemplary embodiment of an apparatus for processing measurement data.

Such an apparatus, according to the embodiment shown in figure 5, includes, a sensor/device profile store 11. This data store 11 may contain a generic list of available sensors/devices and their characteristics (e.g. manufacturer, product name, product ID, data type, data sampling rate, data size per measurement, data format etc). This information may be used by an expected result manager 20 when it is building an EDP.

The apparatus also includes a user profile store 12. This user profile store 12 contains information about device user (e.g. user identification (ID), patient name), his/her measurement information (e.g. choice of devices, frequency of measurement, timing of measurement, reference readings), and medical thresholds. These are the initial inputs from doctor(s) when a patient and his/her sensors/devices are registered. The user profile may be modified when there is any update on the information later on.

In particular, the sensor/device profile of a patient/user may be maintained and updated in line with the update and re-configuration of the sensors/devices by professionals, i.e. doctors and trained nurses. This includes maintenance of the state of the sensors/devices (i.e. active or inactive) associated with a patient/user. Note other factors may have impact on the sensor/device profile, for example the change of the transport network condition may trigger a change of an affected sensor/device profile temporarily.

In addition, a patient's historical data may be included in his/her user profile as part of the initial inputs. Moreover the received measurement data in the received data store may also be included in the user profile, so that they may be used in order to build on historical data correlation for an EDP.

The apparatus according to this embodiment also includes an expected result manager 20. This component 20 creates the EDP which may be used by a data analyzer 30 to check if the data received are as expected or not. The parameters in the EDP (such as frequency of data measured, expected volume of data per transmission, timing of data receiving, etc.) are calculated by using device data available in the device profile store 11, and user measurement information and medical thresholds available in user profile store 12 for each patient/user.

The apparatus according to this embodiment also includes a data analyzer 30. By comparing the data received and parameters in an EDP (stored in expected result manager), this component 30 makes decisions on which tag is assigned to the (or part of the) incoming data. The mechanism which is explained in the flowchart in figure 4 may be implemented by this analyzer 30.

The apparatus according to this embodiment also includes an alert generator 40. Based on the decisions made by the data analyzer 30, this component generates tags/alerts. Before sending the alert, the alert generator 40 may take a number of steps to ensure that data is not received because of other reasons, for example, reach-ability issues, to reduce the potential of a false alarm. For example, if there is network problem (e.g. not very good coverage, or low data rate due to network congestion) the expected measurement data may not be received or not received correctly based on the measurement report configuration of the sensors/devices. Alerts can be sent to patients/clinical staff/IT support/family members etc., depending on for example the nature of the alerts and particular user settings.

The apparatus according to this embodiment also includes a received data store 13. All measurement data which have been received from the users may be stored in this received data store 13. The contents of the data store 13 are analysed by the data analyzer 30 in order to implement the tagging scheme. The data is also forwarded to expected result manager 20 in order to build historical data correlation for EDP.

This invention provides a novel Cloud based remote calibration and tagging scheme for healthcare measurements collected from the sensors/devices of a patient/user of the healthcare services, which is to : 1) identify the incorrect data readings in the collected measurements by comparing them with the Expected Data Profile (EDP) of a patient based on the statistics of the measurements data and the data correlation from a patient's own historical data; 2) further mark the received data with appropriate tag(s) in order to provide more information for the doctors/trained nurses to make professional decisions.

The method and apparatus as described hereinbefore are highly beneficial in order to build a reliable remote healthcare system, which is able to detect the errors in measurement data collection automatically and to notify the errors to the patients and/or the doctors.

Moreover, some of the measurement parameters can also be used to create new tag information to indicate the reliability level of the measurement data. This scheme can help to build up the trust of remote healthcare system from both doctors and patients, thus saving time and effort for both parties, and therefore achieve cost reduction of healthcare.

## Claims

1. A computer-implemented method of processing measurement data received from a remote device monitoring a subject, the method comprising:
comparing the measurement data against an expected data profile (EDP) for the subject, the EDP being derived from historical measurement data and comprising configuration information as to the configuration of the measurement data, and expected value information as to the expected values of the measurement data;
inputting measurement data relating to different health readings and adding the input measurement data to the EDP to form an overall EDP which takes into consideration the different health readings and any data correlation between the different health readings;
marking the measurement data with an error tag if it does not match the configuration information;
marking the measurement data with an abnormal tag if it does not match the data correlation maintained in the EDP and included in the expected value information; and
assessing any measurement data not marked with an error tag or abnormal tag, by excluding the measurement data marked with an error tag or abnormal tag from the assessment, to check whether it indicates a health condition change of the subject, wherein
the configuration information indicates the device identity and/or frequency of receipt and/or amount and/or type of the management data and/or data size per transmission.

2. The method according to claim 1, further comprising:
accepting expert input when creating or updating the EDP and/or when assessing whether the measurement data indicates a health condition change of the subject.

3. The method according to claim 1 or claim 2, further comprising:
marking the measurement data with a health condition change tag if the measurement data indicates a health condition change.

4. The method according to claim 3, wherein
the health condition change tag indicates a type of the health condition change.

5. The method according to any preceding claim, further comprising:
if the measurement data indicates a health condition change, generating an alert indicating the health condition change of the subject.

6. The method according to claim 5, further comprising:
transmitting the alert, wherein the transmission destination is selected in dependence upon the nature of the health condition change and/or a user setting.

7. The method according to any preceding claim, wherein
the EDP includes configuration information and/or expected value information in the form of a range; and
the measurement data must fall within the range to match the configuration information or the expected value information.

8. The method according to any preceding claim, further comprising:
recalibrating the configuration information and/or the expected value information based on the measurement data.

9. The method according to claim 7, further comprising:
recalibrating the configuration information and/or the expected value information, wherein the recalibrating involves updating the range by taking the measurement data into account.

10. The method according to any preceding claim, further comprising:
adding the measurement data to the historical data from which the expected data profile is derived.

11. The method according to any preceding claim, further comprising:
inputting measurement data from an expert and adding the input measurement data to the EDP.

12. The method according to any preceding claim, further comprising:
performing diagnostics on measurement data marked with an abnormal tag to determine a type of abnormality.

13. An apparatus for processing measurement data received from a remote device monitoring a subject, the apparatus comprising:
a memory storing an expected data profile (EDP) for the subject, the EDP being derived from historical measurement data;
an expected result manager configured to compare the measurement data against the EDP, the EDP comprising configuration information as to the configuration of the measurement data, and expected value information as to the expected values of the measurement data for that subject, and to input measurement data relating to different health readings and add the input measurement data to the EDP to form an overall EDP which takes into consideration the different health readings and any data correlation between the different health readings;
a tag generator configured to mark the measurement data with an error tag if it does not match the configuration information, and mark the measurement data with an abnormal tag if it does not match the data correlation maintained in the EDP and included in the expected value information; and
a data analyser configured to assess any measurement data not marked with an error tag or abnormal tag, by excluding the measurement data marked with an error tag or abnormal tag from the assessment, to check whether it indicates a health condition change of the subject, wherein
the configuration information indicates the device identity and/or frequency of receipt and/or amount and/or type of the management data and/or data size per transmission.

## Patentansprüche

1. Computer-implementiertes Verfahren zur Verarbeitung von Messdaten, die von einem entfernten Gerät empfangen werden, welches ein Subjekt überwacht, wobei das Verfahren Folgendes umfasst:
Vergleichen der Messdaten mit einem erwarteten Datenprofil (EDP - Expected Data Profile) für das Subjekt, wobei das EDP aus historischen Messdaten abgeleitet ist und
Konfigurationsinformationen hinsichtlich der Konfiguration der Messdaten und Informationen zu erwarteten Werten hinsichtlich der erwarteten Werte der Messdaten umfasst;
Eingeben von Messdaten bezogen auf unterschiedliche Gesundheitsmesswerte und Hinzufügen der eingegebenen Messdaten zu dem EDP zum Bilden eines Gesamt-EDP, welches die unterschiedlichen Gesundheitsmesswerte und jegliche Datenkorrelation zwischen den unterschiedlichen Gesundheitsmesswerten berücksichtigt;
Kennzeichnen der Messdaten mit einem Fehler-Tag, wenn sie nicht mit den Konfigurationsinformationen übereinstimmen;
Kennzeichnen der Messdaten mit einem Abnormal-Tag, wenn sie nicht mit der Datenkorrelation übereinstimmen, die in dem EDP gepflegt wird und in den Informationen zu erwarteten Werten enthalten ist; und
Bewerten jeglicher Messdaten, die nicht mit einem Fehler-Tag oder einem Abnormal-Tag **gekennzeichnet sind, durch** Ausschließen der Messdaten, die mit einem Fehler-Tag oder einem Abnormal-Tag gekennzeichnet sind, aus der Bewertung, um zu prüfen, ob sie eine Änderung des Gesundheitszustands des Subjekts angeben, wobei
die Konfigurationsinformationen die Geräteidentität und/oder Empfangshäufigkeit und/oder Menge und/oder Art der Managementdaten und/oder Datengröße pro Übertragung angeben.

2. Verfahren nach Anspruch 1, welches ferner Folgendes umfasst:
Akzeptieren einer Experteneingabe beim Erstellen oder Aktualisieren des EDP und/oder beim Bewerten, ob die Messdaten eine Änderung des Gesundheitszustands des Subjekts angeben.

3. Verfahren nach Anspruch 1 oder 2, welches ferner Folgendes umfasst:
Kennzeichnen der Messdaten mit einem Änderung-des-Gesundheitszustands-Tag, wenn die Messdaten eine Änderung des Gesundheitszustands angeben.

4. Verfahren nach Anspruch 3, wobei
der Änderung-des-Gesundheitszustands-Tag eine Art der Änderung des Gesundheitszustands angibt.

5. Verfahren nach einem der vorhergehenden Ansprüche, welches ferner Folgendes umfasst:
wenn die Messdaten eine Änderung des Gesundheitszustands angeben, Erzeugen einer Warnung, welche die Änderung des Gesundheitszustands des Subjekts angibt.

6. Verfahren nach Anspruch 5, welches ferner Folgendes umfasst:
Übertragen der Warnung, wobei das Übertragungsziel in Abhängigkeit von der Natur der Änderung des Gesundheitszustands und/oder einer Benutzereinstellung ausgewählt wird.

7. Verfahren nach einem der vorhergehenden Ansprüche, wobei
das EDP Konfigurationsinformationen und/oder Informationen zu erwarteten Werten in Form eines Bereichs enthält; und
die Messdaten innerhalb des Bereichs fallen müssen, um mit den Konfigurationsinformationen oder den Informationen zu erwarteten Werten übereinzustimmen.

8. Verfahren nach einem der vorhergehenden Ansprüche, welches ferner Folgendes umfasst:
Neukalibrieren der Konfigurationsinformationen und/oder der Informationen zu erwarteten Werten basierend auf den Messdaten.

9. Verfahren nach Anspruch 7, welches ferner Folgendes umfasst:
Neukalibrieren der Konfigurationsinformationen und/oder der Informationen zu erwarteten Werten, wobei das Neukalibrieren ein Aktualisieren des Bereichs durch Berücksichtigung der Messdaten beinhaltet.

10. Verfahren nach einem der vorhergehenden Ansprüche, welches ferner Folgendes umfasst:
Hinzufügen der Messdaten zu den historischen Daten, aus welchen das erwartete Datenprofil abgeleitet ist.

11. Verfahren nach einem der vorhergehenden Ansprüche, welches ferner Folgendes umfasst:
Eingeben von Messdaten von einem Experten und Hinzufügen der eingegebenen Messdaten zu dem EDP.

12. Verfahren nach einem der vorhergehenden Ansprüche, welches ferner Folgendes umfasst:
Durchführen einer Diagnose an Messdaten, die mit einem Abnormal-Tag gekennzeichnet sind, um eine Art der Abnormalität zu bestimmen.

13. Vorrichtung zur Verarbeitung von Messdaten, die von einem entfernten Gerät empfangen werden, welches ein Subjekt überwacht, wobei die Vorrichtung Folgendes umfasst:
einen Speicher, der ein erwartetes Datenprofil (EDP - Expected Data Profile) für das Subjekt speichert, wobei das EDP von historischen Messdaten abgeleitet ist;
einen Manager für erwartete Ergebnisse, der konfiguriert ist zum Vergleichen der Messdaten mit dem EDP, wobei das EDP Konfigurationsinformationen hinsichtlich der Konfiguration der Messdaten und Informationen zu erwarteten Werten hinsichtlich der erwarteten Werte der Messdaten für das Subjekt umfasst, und zum Eingeben von Messdaten bezogen auf unterschiedliche Gesundheitsmesswerte und Hinzufügen der eingegebenen Messdaten zu dem EDP zum Bilden eines Gesamt-EDP, welches die unterschiedlichen Gesundheitsmesswerte und jegliche Datenkorrelation zwischen den unterschiedlichen Gesundheitsmesswerten berücksichtigt;
einen Tag-Generator, der konfiguriert ist zum Kennzeichnen der Messdaten mit einem Fehler-Tag, wenn sie nicht mit den Konfigurationsinformationen übereinstimmen, und Kennzeichnen der Messdaten mit einem Abnormal-Tag, wenn sie nicht mit der Datenkorrelation übereinstimmen, die in dem EDP gepflegt wird und in den Informationen zu erwarteten Werten enthalten ist; und
einen Datenanalysator, der konfiguriert ist zum Bewerten jeglicher Messdaten, die nicht mit einem Fehler-Tag oder einem Abnormal-Tag **gekennzeichnet sind, durch** Ausschließen der Messdaten, die mit einem Fehler-Tag oder einem Abnormal-Tag gekennzeichnet sind, aus der Bewertung, um zu prüfen, ob sie eine Änderung des Gesundheitszustands des Subjekts angeben, wobei
die Konfigurationsinformationen die Geräteidentität und/oder Empfangshäufigkeit und/oder Menge und/oder Art der Managementdaten und/oder Datengröße pro Übertragung angeben.

## Revendications

1. Procédé mis en œuvre par ordinateur pour traiter des données de mesure reçues d'un dispositif distant surveillant un sujet, le procédé comprenant les étapes consistant à :
comparer les données de mesure à un profil de données attendu (EDP) pour le sujet, l'EDP étant dérivé de données de mesure historiques et comprenant des informations de configuration quant à la configuration des données de mesure, et des informations de valeur attendue quant aux valeurs attendues des données de mesure ;
entrer des données de mesure relatives à différentes lectures de santé et ajouter les données de mesure d'entrée à l'EDP pour former un EDP global qui prend en considération les différentes lectures de santé et toute corrélation de données entre les différentes lectures de santé ;
marquer les données de mesure avec une étiquette d'erreur si elles ne correspondent pas aux informations de configuration ;
marquer les données de mesure avec une étiquette anormale si elles ne correspondent pas à la corrélation de données maintenue dans l'EDP et incluse dans les informations de valeur attendue ; et
évaluer de quelconques données de mesure non marquées avec une étiquette d'erreur ou une étiquette anormale, en excluant les données de mesure marquées avec une étiquette d'erreur ou une étiquette anormale à partir de l'évaluation, pour vérifier si cela indique un changement d'état de santé du sujet, dans lequel
les informations de configuration indiquent l'identité du dispositif et/ou la fréquence de réception et/ou la quantité et/ou le type des données de gestion et/ou une taille de données par transmission.

2. Procédé selon la revendication 1, comprenant en outre l'étape consistant à :
accepter une entrée d'expert lors d'une création ou d'une mise à jour de l'EDP et/ou lors de l'évaluation que les données de mesure indiquent ou non un changement d'état de santé du sujet.

3. Procédé selon la revendication 1 ou la revendication 2, comprenant en outre l'étape consistant à :
marquer les données de mesure avec une étiquette de changement d'état de santé si les données de mesure indiquent un changement d'état de santé.

4. Procédé selon la revendication 3, dans lequel l'étiquette de changement d'état de santé indique un type du changement d'état de santé.

5. Procédé selon l'une quelconque des revendications précédentes, comprenant en outre l'étape consistant à :
si les données de mesure indiquent un changement d'état de santé, générer une alerte indiquant le changement d'état de santé du sujet.

6. Procédé selon la revendication 5, comprenant en outre l'étape consistant à :
transmettre l'alerte, dans lequel la destination de transmission est sélectionnée en fonction de la nature du changement d'état de santé et/ou d'un paramètre d'utilisateur.

7. Procédé selon l'une quelconque des revendications précédentes, dans lequel
l'EDP comprend des informations de configuration et/ou des informations de valeur attendue sous la forme d'une plage ; et
les données de mesure doivent se situer dans la plage pour correspondre aux informations de configuration ou aux informations de valeur attendue.

8. Procédé selon l'une quelconque des revendications précédentes, comprenant en outre l'étape consistant à :
recalibrer les informations de configuration et/ou les informations de valeur attendue sur la base des données de mesure.

9. Procédé selon la revendication 7, comprenant en outre l'étape consistant à :
recalibrer les informations de configuration et/ou les informations de valeur attendue, dans lequel le recalibrage implique une mise à jour de la plage en tenant compte des données de mesure.

10. Procédé selon l'une quelconque des revendications précédentes, comprenant en outre l'étape consistant à :
ajouter les données de mesure aux données historiques à partir desquelles le profil de données attendu est dérivé.

11. Procédé selon l'une quelconque des revendications précédentes, comprenant en outre l'étape consistant à :
entrer des données de mesure d'un expert et ajouter les données de mesure d'entrée à l'EDP.

12. Procédé selon l'une quelconque des revendications précédentes, comprenant en outre l'étape consistant à :
effectuer des diagnostics sur des données de mesure marquées d'une étiquette anormale pour déterminer un type d'anomalie.

13. Appareil pour traiter des données de mesure reçues d'un dispositif distant surveillant un sujet, l'appareil comprenant :
une mémoire stockant un profil de données attendu (EDP) pour le sujet, l'EDP étant dérivé de données de mesure historiques ;
un gestionnaire de résultats attendus configuré pour comparer les données de mesure à l'EDP, l'EDP comprenant des informations de configuration quant à la configuration des données de mesure, et des informations de valeur attendues quant aux valeurs attendues des données de mesure pour ce sujet, et pour entrer des données de mesure relatives à différentes lectures de santé et ajouter les données de mesure d'entrée à l'EDP pour former un EDP global qui prend en considération les différentes lectures de santé et toute corrélation de données entre les différentes lectures de santé ;
un générateur d'étiquette pour marquer les données de mesure avec une étiquette anormale si elles ne correspondent pas aux informations de configuration, et marquer les données de mesure avec une étiquette anormale si elles ne correspondent pas à la corrélation de données maintenue dans l'EDP et incluse dans les informations de valeur attendue ; et
un analyseur de données pour évaluer de quelconques données de mesure non marquées avec une étiquette d'erreur ou une étiquette anormale, en excluant les données de mesure marquées avec une étiquette d'erreur ou une étiquette anormale à partir de l'évaluation, pour vérifier si cela indique un changement d'état de santé du sujet, dans lequel
les informations de configuration indiquent l'identité du dispositif et/ou la fréquence de réception et/ou la quantité et/ou le type des données de gestion et/ou une taille de données par transmission.
